Europäisches Patentamt

European Patent Office

Office européen des brevets

(11) Publication number: **0 548 410 A1**

# EUROPEAN PATENT APPLICATION

(21) Application number: **91203382.6**

(22) Date of filing: **23.12.91**

(51) Int. Cl.5: **A23L 1/23**, A23L 1/318, C12N 1/00

(43) Date of publication of application:
**30.06.93 Bulletin 93/26**

(84) Designated Contracting States:
**AT BE CH DE DK ES FR GB GR IT LI LU NL SE**

(71) Applicant: **UNILEVER N.V.**
**Weena 455**
**NL-3013 AL Rotterdam(NL)**

(72) Inventor: **Bosse, Michael Antonie, c/o Unilever Research**
**Vlaardingen Lab., Olivier van Noortlaan 120**
**3133 AT Vlaardingen(NL)**
Inventor: **Rhee, Renee van, c/o Unilever Research**
**Vlaardingen Lab., Olivier van Noortlaan 120**
**3133 AT Vlaardingen(NL)**
Inventor: **Straaten, Jan Philip van, c/o Unilever Research**
**Vlaardingen Lab., Olivier van noortlaan 120**
**3133 AT Vlaardingen(NL)**

(74) Representative: **Roscoe, Brian Corrie et al**
**UNILEVER PLC Patents Division Colworth House Sharnbrook**
**Bedford MK44 1LO (GB)**

(54) **Process for producing edible matter having an enhanced flavour, a flavouring substance obtainable according to said process and the use of such a flavouring substance for imparting flavour to foodstuffs.**

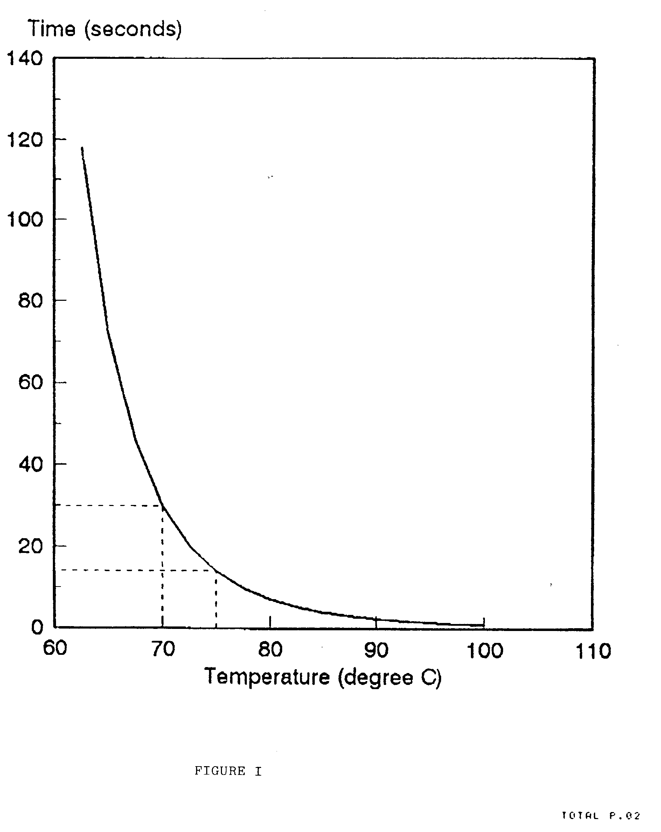

(57) The invention relates to a process for producing edible matter having an enhanced flavour derived from incubating a microorganism capable of producing edible non toxic substances in an aqueous fermentation medium with an $a_w$ of at least 0.97 in the presence of meat protein and a carbon containing source of energy, wherein said microorganism is cultivated in said aqueous fermentation medium for a period of 1 to 20 hours after finishing the logarithmic growth phase, i.e. after 0-20 hours in the stationary growth phase. After sufficient fermentation the microorganism can be inactivated in such a manner that the character of the flavour obtained from the fermentation is not altered. Such inactivation can be achieved by heating the fermentation broth during a period of time t at a temperature T wherein the values of t and T are equal to or lower than the values illustrated in the curve of figure 1.

EP 0 548 410 A1

The present invention relates to a process for producing edible matter having an enhanced flavour derived from incubating a microorganism capable of producing edible non-toxic substances in an aqueous fermentation medium in the presence of meat protein and a carbon containing source of energy. The invention also relates to a meat flavouring substance obtained by said process and to a method of flavouring or aromatizing foodstuffs, using the flavouring substance according to the present invention. In particular the invention is directed at a process wherein said enhanced flavour is an enhanced raw meat flavour.

Microorganisms are known to play an important role in the flavour production of dry sausages of for example the salami type. Generally spoken these microorganisms can be categorized as follows: Microorganisms involved in the production of acids from carbohydrates (Lactobacilli, Pediococci), microorganisms involved in the breakdown of proteins to free lower peptides or amino acids (Micrococci, yeasts and moulds) and micro-organisms involved in the breakdown of fat (Micrococci, yeasts and moulds). Traditionally, the fermentation of these products relied on the microorganisms normally present in the raw material, however, the use of starter cultures as a means of controlling the process and of ensuring quality has become widespread, particularly over the past twenty years. The starter cultures available for example for raw meat products are lactic acid bacteria, yeasts and moulds. A review of current practice has been given in "Die Fleischwirtschaft", Volume 67, No. 3 (1987), pages 303-314 by F.K. Lücke and H. Hechelmann.

The success of the use of starter cultures for the manufacture of fermented raw meat products depends on a rather large number of factors, however, and sometimes the optimal requirements to be met at the same time are conflicting. Obtaining the same flavouring and aromatizing effect could be beneficial if this could be reached by use of an aromatizing substance. Hence there is still a need for an aromatizing or flavouring substance which upon incorporation into raw meat products gives rise to the typical fermented raw meat flavour and aroma.

The present invention aims at the provision of such a flavouring or aromatizing material using a fermentation process, so that the required material is produced by a "natural route" and does not need the use of "synthetic" products in order to arrive at the desired flavour or aroma, when applied in foodstuffs especially in fermented raw meat products such as dry sausage.

In "Die Nahrung", 27, 9, (1983), pages 831-836 (Gadjeva, D. et al) and "Die Nahrung", 30, 8, (1986), pages 829-832 (Miteva, E. et al) the influence of addition of a Candida utilis yeast culture to two sorts of Loukanka type raw dried Bulgarian sausage (smoked and non-smoked) is discussed. In the process described the yeast strain was cultured and a 3 liter sample of said culture with a titer of $10^9$ - $10^{10}$ cells/ml was added to 100 kg of meat mass which was subsequently allowed to ripen. Both the smell and taste of the resulting sausages was improved. The maximum growth of the yeasts resulted in formation of larger amounts of total carbonyls, monocarbonyls and their subfractions, and initiated a process of intensive hydrolysis of lipids which resulted in significant differences in fat acidity. The more intensive lipolysis caused an increase in the specific flavour characteristic of the sausages. The addition of C. utilis led to higher amounts of saturated and unsaturated aldehydes, especially in the non-smoked sausages.

The flavouring obtained in such a manner has the disadvantage that it can only be used in the production of sausages produced from the solid meat medium in which said microorganism is cultivated. Furthermore the amount of time required for said flavouring to develop can take anything from 10 to 30 days. A further problem resides in the difficulty of mixing a solid mass in order to obtain a uniform flavour distribution.

In EP-A-0,357,812 a process is disclosed for improving the flavour of protein products derived from microorganisms which comprises culturing the microorganism in the presence of a flavour enhancing additive and heat treating the fermentation broth in order to enhance the flavour. The flavour enhancing additive is derived from either an edible animal by-product or fatty acids whereby said animal by-product is present from 0.5 - 5 wt/vol.%. The process can be carried out with microorganisms capable of producing edible non-toxic proteins, preferably yeast, especially belonging to any of the genera Candida, Hansenula or Debaryomyces. It is preferred to heat the fermentation broth to a temperature of 70-90 °C for t = 2-20 minutes or more, preferably for t = 2-20 at T = 80-85 °C. In said process the specific flavouring and aroma are obtained by the heating step. The known process enables uniform distribution of the flavouring substances and provides a medium from which said flavouring substances can be extracted through various means known to an expert in this field as the flavouring substances are produced in a fluid medium.

Surprisingly it was found that such a process for producing edible matter having an enhanced flavour derived from incubating a micro-organism capable of producing edible non-toxic substances in an aqueous fermentation medium with an $a_w$ of at least 0.97 in the presence of meat protein and a carbon containing source of energy could be improved by cultivating said microorganism in said aqueous fermentation medium for a period of 1 to 20 hours after finishing the logarithmic growth phase, i.e. after 0-20 hours in the stationary phase.

The process according to the invention differs from the process mentioned in EP-A-0,357,812. The flavour in the process mentioned in EP-A-0,357,812 is provided by heat treatment during 2-20 minutes at a temperature ranging from 70-99°C, preferably from 80-85°C. Whereas in the subject invention the improved flavour is obtained by cultivating said microorganism in said aqueous fermentation medium for a period of 1 to 20 hours after finishing the logarithmic growth phase, i.e. after 0-20 hours in the stationary phase without needing subsequent heat treatment to obtain the desired flavour. The flavour obtained through the process according to the invention has a different character when compared to the flavour obtained through the process according to EP-A-0,357,812.

Sometimes it is preferable to inactivate the microorganisms contained in the fermentation medium after the desired flavour has been produced. This will be the case for example when the flavour is to be used in a microbiologically controlled process such as the production of dry sausage. The inactivation of the microorganisms must be carried out in such a manner that the character of the flavour resulting from the fermentation process according to the invention is not altered. This can for example be achieved by inactivating said microorganism after sufficient fermentation by heating the fermentation broth during a period of time t at a temperature T, whereby the values of t and T remain below or are equal to the values illustrated in the curve of figure 1. The values of t and T are selected in such a manner that the inactivation through heating for certain periods of time at certain temperatures can give rise to a fermented raw meat flavour, in contrast to the process of EP-A-0,357,812 in which an improved savoury flavour is obtained only after heating for 2-20 minutes at temperatures between 70-99°C. The use of milder conditions for inactivation in the preferred process according to the invention wherein the microorganisms are inactivated apparently prevents the loss of components giving the specific flavour and aroma obtainable through the fermentation process according to the invention, in particular components leading to a fermented raw meat flavour. In EP-A-0,357,812 the use of harsher inactivation conditions in fact leads to the flavour obtained in said known process, which has a different character than the flavour obtained through the process according to the invention.

The process according to the invention does not only have the advantages of uniform distribution of flavouring substances and provision of a medium from which said flavouring substances can be extracted through various means known to an expert in this field as has already been described for the process according to EP-A-0,357,812 but also has other unexpected advantages.

The process according to the invention has the additional advantage that it results in a fermentation medium containing flavouring in a con centrated form. The use of as little as 1% by weight of the fermentation medium obtained with the process according to the invention is for example adequate to provide soups and other edible substances with sufficient flavour. The process according to the invention leads to an unexpected high concentration of flavour and aroma.

A further advantage of said process lies in the short time required to obtain said concentrated flavour, namely 0-20 hours after the microorganisms have finished the logarithmic growth phase.

In a preferred embodiment of the process according to the invention, i.e. if the resulting flavour is to be applied in a microbiologically controlled process, the microorganisms are preferably inactivated during a period of time selected from between 14 and 30 seconds at a temperature equal to or lower than the value illustrated in the curve of Fig. 1 corresponding to said inactivation period (i.e. any combination of values for t and T lying in the area below the curve or on the curve and not lying outside the dotted lines can be selected for inactivation of the microorganisms in a preferred embodiment of the method according to the invention). Therefore the process according to the invention is preferably carried out in such a manner that the microorganisms are inactivated during a period of time at a temperature equal to or below 75°C. Conditions for use in the process according to the invention leading to good results are heating up to 70°C for 30 seconds and heating up to 75°C for 14 seconds. Good results were also obtained from heating at 65°C for 30 seconds. Analysis of the curve in Fig. 1 will reveal the various combinations of heating time and maximum temperatures that are possible for obtaining good results with the preferred process according to the invention. In general any combination of t and T situated on or below the curve of Fig. 1 is suitable for use in the inactivation of microorganisms in a preferred process according to the invention.

The fermentation medium to be used in the process according to the invention can be any suitable fermentation medium, but is preferably meat or a meat extract such as beef extract in an aqueous solution. Fermentation medium comprising from about 2 to about 3 % by weight of meat or meat extract and from 1.5-2.5 % by weight of a carbon containing source of energy has been found suitable for use in the process according to the invention. A suitable carbon containing source of energy can be a sugar or a mixture of sugars, preferably selected from the group consisting of glucose, lactose and maltose. An especially suitable fermentation medium for use in a process according to the invention comprises 2.5 % by weight of beef extract, 2.0 % by weight of glucose and 2.0 % by weight of sodium chloride all dissolved in 1 litre of distilled water. Said culture medium is referred to as BM-8 and flavour obtained from fermentation medium resulting from the process according to the invention wherein said culture medium is fermented by the yeast C. utilis CBS 7502 (deposited at the CBS in Baarn, the Netherlands) is referred to as the BM-8 flavour block.

It is of particular interest that the method according to the invention in which a liquid fermentation medium is used can lead to a fermented raw meat flavour, in particular a dry sausage flavour. Until now it has not been possible to obtain a fermented raw meat flavour using a liquid medium.

The process according to the invention can be carried out using a microorganism selected from the group comprising bacteria yeasts and moulds. Suitable yeasts can be selected from one of the genera Candida and Hansenula for example. It has been discovered that strains selected from the species Candida utilis such as the strain Candida utilis CBS 7502 or Candida utilis ATCC 26387 are especially suited for use in a process according to the invention. A strain selected from the species Debaryomyces hansenii such as the strain Debaryomyces hansenii CBS 7531 is also suitable for use in a process according to the invention. All strains of C. utilis and D. hansenii that were tested contributed to the flavour of dry sausage: a fermented sausage contribution was obtained with C. utilis CBS 7502 and MY 87, a fermented sausage salami-like contribution of somewhat other character was obtained with D. hansenii CBS 7531 and an excellent very salami-like contribution was obtained with C. utilis ATCC 26387 when used in a process according to the invention wherein the fermentation medium was BM8. In Example 5 results are given of organoleptical evaluation of flavour obtained using the process according to the invention whereby various strains of C. utilis and D. hansenii were used with fermentation medium BM8. C. utilis strains were incubated at 30°C and D. hansenii strains were incubated at 25°C.

The fermentation in the process according to the invention is preferably carried out under aerobic conditions i.e. that during fermentation oxygen, preferably in the form of air is passed through the aqueous fermentation broth. It has been found beneficial to have a dissolved oxygen tension equivalent to at least 10% during the fermentation. The dissolved oxygen tension is expressed as a percentage of the amount of oxygen that is dissolved in the solution when the saturation value is reached at atmospheric pressure.

The temperature applied during the cultivation of the microorganism in the process according to the invention is dependent on the type of microorganism used but will suitably be in the range of 20°C to 30°C, preferably from 25°C to 35°C. Preferably C. utilis strains are incubated at 30°C and D. hansenii strains are incubated at 25°C. Changing the temperature in the range of 25°C to 35°C does not appear to affect the flavour quality. Only the fermentation time is affected. When the temperature is increased a decrease is observed for the required fermentation time.

The pH value of the fermentation medium during the cultivation is not critical to the process but can be of influence for the flavour obtained and fermentation of the process according to the invention can preferably be adjusted to a value between 4.5 and 6.0, with more preference for a pH of about 5.0. The adjustment can be achieved by adding inocuous acids such as lactic acid, phosphoric acid and the like. The use of lactic acid for this purpose is preferred.

As it had been reported that the proteolytic activity of lactobacilli decreases with increasing NaCl concentrations ( G.M. Vignolo, A. Pesce de Ruis Holgado and G. Oliver, J. Food protection vol. 51, no. 6., 1988) the effect of various NaCl concentrations on flavour development in the process according to the invention was evaluated (see Example 6).

It was found that flavour developed in the absence of NaCl had a different and interesting character in comparison to flavour developed in the presence of NaCl.

Furthermore it was ascertained that yeast was able to grow well in the presence of NaCl, even when 10% by weight of NaCl was present.

In fact it was discovered that the process according to the invention can benefit from variation in concentration of NaCl in the fermentation medium. Preferred flavours can be obtained in a fermentation medium comprising either 0% or 1,5-2,5 % by weight NaCl in the process according to the invention. An especially preferred flavour is obtained using the process according to the invention when NaCl is present in the fermentation medium in 2% by weight. In particular use of a fermentation medium comprising beef extract, glucose and NaCl which is fermented by yeast according to the process according to the invention leads to a preferred flavour. A suitable example of a preferred flavour is the BM-8 flavour block obtained via fermentation of BM-8 medium by the C. utilis strain CBS 7502. (Said medium has been defined earlier in the specification).

For specific aromatizing purposes yeast extract can be incorporated into the culture medium to be used according to the invention. Yeast extract is known to be a protein source with typical "animal" notes. Addition of yeast extract to the culture medium, preferably in 0.5 or 1%, in particular at a level of about 0.5 % by weight can improve the flavour obtained through the process according to the invention. Yeast extract from Fould Springer was found to be especially beneficial to the flavour. In particular the addition to the BM-8 medium led to an improved flavour which was eminently suited for use in a broad area of food products such as fresh sausages, especially dry type sausages, snacks, meals, sauces, spreads and soups (see Example 7).

The incorporation of yeast extract also results in a higher growth rate and in a higher dry weight content and viable count of the fermentation broth. The addition of yeast extract under cultivation conditions of $T = 20°\text{-}38°C$ with a pH between 4.5-6.0 and a dissolved oxygen tension of more than 10 % leads to an increase in the growth rate such that the stationary phase is reached in less than 50 hours.

The addition of substances that can serve as precursors for aroma compounds such as phenyl acetic acid, alcohols etc. to a fermentation medium for use in the process according to the invention can also enhance the flavour. Examples of such substances are fusel oils and amino acids.

The addition of amino acids in an amount of 0.3 % to fermentation medium for use in a process according to the invention in most cases results in characteristic flavours.

The flavour impression resulting at pH 5.0 after addition of various amino acids to BM8 media for example was remarkably improved. In particular addition of leucine, phenyl alanine, cysteine, methionine and tryptophane to the fermentation medium remarkedly improved the BM8 flavour. The results are given in Example 8.

The addition of spices to a fermentation medium for use in the process according to the invention can also improve the resulting flavour. It is therefore a preferred embodiment of the invention to include one or more spices in the fermentation medium to be used in the process according to the invention, whereby said spices are preferably selected from spices usually added as ingredients in the preparation of food products.

In the case of sausage preparation for example the following spices are typically used: white pepper, black pepper, nutmeg, clove, ginger powder, piment and garlic powder. The results that were obtained for flavour impression and contribution in dry type sausage using flavour obtained through the process according to the invention with fermentation medium comprising said spices in amounts of 0.1 or 0.5 % are given in Example 9.

Addition of white pepper to a fermentation medium comprising meat extract, glucose and NaCl was found to improve the flavour obtained through the process according to the invention. This was also the case for addition of garlic powder. Such flavours are considered to be very suited for use in preparing dry type sausage. Use of a fermentation medium comprising white pepper and or garlic powder in a process according to the invention can lead to a preferred flavour that is suitable for use in the preparation of food products with a sausage flavour, in particular a fermented raw meat flavour. A fermentation medium suitable for producing such a preferred flavour can be obtained by addition of the mentioned spice or spices to the BM-8 medium and further carrying out the process according to the invention.

The influence of ripening was also evaluated on the flavour obtained using the process according to the invention. Tests were carried out using anaerobic ripening at various temperatures and by addition of a hydrolyzing enzyme such as papaine to achieve cell lysis. Anaerobic ripening resulted in the development of yeasty flavours at 35°C both with and without papaine.

The fermentation broth obtained through a process according to the invention can be submitted to further downstream processing such as spray drying, freeze drying, concentration by vacuum evaporation, reverse osmosis or freeze concentration. The preferred method of downstream processing will depend on the final application of the flavour in the fermentation broth. The selection of a particular method for downstream processing will be obvious to the expert. It is, for example, possible to produce powders from said fermentation broth by various methods well known to an expert. The manner in which the fermentation broth is dried is not particularly critical, but it should not impair the quality of the edible flavouring material it contains.

It was discovered that powders obtained from fermentation liquids obtained from the process according to the invention also gave good results in organoleptic evaluation tests in particular the flavours obtained using fermentation media comprising one or more amino acids selected from the group comprising isoleucine, phenyl alanine and tryptophane. Therefore a preferred process according to the invention for obtaining sausage-like flavour comprises the use of a fermentation medium comprising one or more amino acids selected from the group comprising isoleucine, phenyl alanine and tryptophane.

The present invention also relates to an edible matter with enhanced flavour obtainable through the process according to the invention in the various embodiments described. Furthermore the present invention also relates to a flavouring substance comprising the above mentioned edible matter in particular a flavouring substance exhibiting a fermented meat flavour. Such a meat flavouring substance may comprise other materials such as flavour precursors, flavouring agents, flavour boosters, stabilizers, flow-control agents or anticaking agents, and such like substances which are well known to the expert.

The subject invention also relates to a method for imparting flavour to foodstuffs or ingredients for foodstuffs by incorporating a flavouring or aromatizing amount of the edible matter or the flavouring substance as described above into the foodstuff or ingredient for a foodstuff. Preferably the foodstuff is sausage material when the flavouring substance exhibits a fermented raw meat flavour, however, the flavouring agent can also be incorporated into meals, meal components, snack products, and such like foodstuffs.

The invention is also directed at foodstuffs or ingredients for foodstuffs, that can be obtained by the above mentioned method for imparting flavour.

The invention will now be illustrated by the following examples.

Example 1

The preparation of a fermented (raw) meat flavour, in particular a dry sausage flavour is demonstrated in this example.

100 ml of a substrate containing 25 g beef extract, 20 g NaCl and 20 g glucose/l with a pH 5.8 were inoculated in a 500 ml Erlenmeyer with 1% C. Utilis CBS 7502 subculture and then placed in a shaking incubator at 30°C at a speed of 250-300 rpm for 5 days. The pH increased to above 8.0. After adjustment of the pH to 5.0 with lactic acid, the odour release was pungent sweaty reminiscent of the odour of salami. It was demonstrated that the plain taste of a chemically acidified semi-dried sausage was improved by addition of the fermented substrate as followed: 1 g of fermented substrate was made up with water to 10 g and this was mixed with 40 g of finely cuttered chemically acidified semi-dried sausage. The resulting product was organoleptically evaluated by a skilled panel.

Example 2

Fermentor experiment on 10 l scale

A fermentor run involving a 10 l fermentation followed by downstream processing of the fermentation broth was carried out.

The Candida utilis CBS 7502 strain was used. The inoculum was grown in BM8 (see below).

| BM8 medium | |
| --- | --- |
| Beef extract (ex Anglo or Quest) | 2.5% (w/v) |
| Glucose (ex Duchefa) | 2.0% (w/v) |
| NaCl (ex Merck) | 2.0 (w/v) |
| in tapwater | |

After inoculation a 10 l scale fermentation was carried out at 30°C with a pH of 4.5 and a dissolved oxygen tension of 10% at 1 atm. In approximately 24 hours the glucose substrate was almost completely consumed. After approximately 60 hours of fermentation the yeast entered the stationary phase. Organoleptic evaluation of samples taken during the fermentation indicated that particularly in the first 20 hours of the stationary phase the flavour quality was optimal, especially in the first 10 hours. After 80 hours the broth was pasteurised, for 30" at 65°C using a plate heat exchanger. The product was organoleptically evaluated as described in Example 1.

It was ascertained that in fact heat treatment at higher t and T values than shown in the curve of figure 1 resulted in loss of flavour.

Example 3

In this example it is demonstrated that the flavours generated in the process according to the invention as described in Example 2 could be improved by using a free pH and a different medium.

The production time was drastically reduced to 2-3 days i.e. 0-20 hours after the microorganism had finished the logarithmic growth phase.

The procedure of Example 2 was followed using BM8-1 medium. BM8-1 differs from BM8 medium solely through the addition of yeast extract (ex Fould Springer) in 0.5% (w/e). During the whole fermentation period, pH was set free. The pasteurisation step according to Example 2 was followed by spray-drying and drying on carrier

A Büchi 190 minispraydryer (ex Mettler) was used.

| Conditions: | |
|---|---|
| Air temperature | inlet 180°C. |
| outlet | 100°C. |
| BM8-1 feed | 200 ml/h. |

The dry weight content of the BM8-1 material at the end of the fermentation was 5.2% (w/w). The following materials were added in various combinations to increase the dry weight level:

| Maltodextrin MD 20 | ex Avebe |
|---|---|
| Arabic gum GDMF | ex Quest |
| MCT oil | ex Van den Bergh Foods |
| NaCl | ex Akzo |

After spray drying the product a powder was evaluated in a dry sausage. The powder was added to meat dough for producing a dry sausage in an amount equivalent to 1% of a slurry. The addition led to a specific salamilike flavour.

Example 4

In this example it is demonstrated that certain pH values give rise to different flavour blocks.

The process according to Example 1 was carried out, wherein the fermentation took place at various pH-values that were kept constant during the fermentation process. A pH-value equal to or higher than 4.5 gave good flavour. At pH 4.5 and 5.0 the flavour block was snijworst-like and at pH 6.0 the flavour was different. It was salami-like.

Example 5

The purpose of this example is to show that use of different yeast strains resulted in flavour blocks with different characters.

Using the methods described in Example 1 the flavour of BM8 medium fermented with 4 C. utilis strains and 5 D. hansenii strains were organoleptically evaluated.

C. utilis ATCC 26387 resulted in an excellent pungent sweaty + indol/scatol flavour contribution. D. hansenii CBS 7531 resulted in a very good indol/scatol flavour contribution.

The origin of the strains and the results are presented in Table 1.

Example 6

The purpose of this example is to show the influence on the flavour that NaCl to the substrate formulation had.

BM8 media were prepared according to methods set out in Example 1 wherein NaCl was added to the media in a concentration of 0, 2, 3, 4 and 10 %. The substrates were fermented with C. utilis CBS 7502 using the method as described in Example 1. The products were organoleptically evaluated on flavour impression and taste contribution in dry sausage pulp. The taste contribution is given in Table 2.

Example 7

The purpose of this example is to show that addition of yeast extract bone meal and fusel alcohol to the fermentation substrate resulted in flavour blocks with different characters.

BM8 substrates according to Example 1 were prepared with the addition of 4 yeast extracts and 2 bone meals from different suppliers in amounts of 0.5% and 1%.

Using the method described under Example 1 the flavour production with C. utilis CBS 7502 was carried out and the products were organoleptically evaluated.

7

The results are shown in Table 3.

Example 8

The purpose of this example is to show that addition of amino-acids to the fermentation substrate resulted in flavour blocks with different characters.

BM8 media were prepared according to the method described under Example 1 and a concentration of 0.3 % was applied for all amino acids to be tested. The substrates were fermented at 30 °C with C. utilis CBS 7502 as described in Example 1 and the flavour impression in the final products was evaluated using the method also described in Example 1.

The results are shown in Table 4.

Example 9

The purpose of this example is to show that addition of spices to a fermentation substrate resulted in flavour blocks with different characters.

BM8 was prepared as described in Example 1 and various media were obtained by addition of 0.5 % white pepper, bleach pepper and garlic powder respectively and 0.1 % garlic powder. The flavour production according to Example 1 was carried out in these substrates with C. utilis CBS 7502.

The flavour impression at pH 5.0 and the flavour contribution in dry sausage was determined according to the methods also described in Example 1.

The results are given in Table 5.

## TABLE 1

Organoleptical evaluation of flavours produced by
C. Utilis and D. hansenii strains in BM 8 medium

| Yeast strain | Taste contribution in sausage pulp (2% dosage) |
|---|---|
| C. utilis CBS 7502 | positive |
| " MY 87 | positive |
| " MY 157 | weakly positive |
| " ATCC 26387 | excellent |
| D. hansenii H2* Wiesby | positive but weak |
| " 6 salami | positive but weak |
| " CBS 7531 | good contribution |
| " 14 | weak |
| " 16 | weak |
| " 18 | weak |

* C. utilis strains: 5 days incubation at 30°C
  D. hansenii strains: 7 days at 25°C


## TABLE 2

Influence of the NaCl concentration in BM8 substrate on the
growth and flavour formation by C. utilis CBS 7502 at 25°C.
Taste contribution in dry sausage pulp.

| | Taste contribution in dry sausage pulp |
|---|---|
| 0% NaCl | positive, different character |
| 1% " | positive |
| 2% " | very positive |
| 3% " | weakly positive |

## TABLE 3

Effect of yeast extract, bone meal and fusel oil on the flavour production of C. utilis CBS 7502 in BM8 at 25°C

| No. | Compound added to BM8 medium | % | Taste contribution in sausage pulp (2% dosage) |
|---|---|---|---|
| 1 | No addition | | positive |
| 2 | Yeast extract Fould Springer | 0.5 | "      + something extra |
| 3 | | 1.0 | "   +   "    " |
| 4 | Gistex Gist Brocades KGV502 | 0.5 | "      + slightly " |
| 5 | | 1.0 | "   +   "    " |
| 6 | Yeast extra YEP Quest 6069274 | 0.5 | " |
| 7 | Malted yeast Tefco | 0.5 | " |
| 8 | | 1.0 | " |
| 9 | Bone protein Marrow G25-2 | 0.5 | "      + slightly extra |
| 10 | | 1.0 | "   +   "    " |
| 11 | Bone protein B070658 | 0.5 | "   +   "    " |
| 12 | | 1.0 | "   +   "    " |
| 13 | Methyl-butanol-2 | 0.1 | not negative |

TABLE 4

Influence of amino acids on the flavour production pf C. utilis CBS 7502 in BM8-medium at 30°C. pH rate, flavour impression at pH 5.0 and taste contribution in sausage pulp of end products.

| BM8 + amino acids | Flavour impression | T a s t e contribution in sausage pulp |
|---|---|---|
| 1 No amino acid | sweaty | +[2] |
| 2 Glycine (S) [2] | weakly yeasty acid | ++ |
| 3 L-Leucine (M) | typical, alcohol, ester | ++ |
| 4 Isoleucine (M) | pungent, alcohol, ester | ++ |
| 5 Phenylalanine (F) | strong pungently sweaty | +++ |
| 6 L-Cysteine HCl (M) | sweaty, sulphur | +++ |
| 7 DL-Methionine (S) | strong methional | ++ |
| 8 DL-Tryptophane (M) | indol/skatol | +++ |
| 9 L-Asparagine (M) | fresh yeasty | ++ |
| 10 L-Arginine (M) | fresh yeasty, acid | ++ |

[1]) S = Sigma; M = Merck; F = Fluka

[2]) + = positive contribution; +++ = strong contribution

TABLE 5

Influence of spices on the flavour formation of C. utilis CBS 7502 in BM8 substrate at 30°C.
pH rate and taste contribution in sausage pulp.

| Spice | conc.% | Taste contribution in sausage pulp |
|---|---|---|
| Bleach pepper | 0.5 | weakly positive |
| White pepper | 0.5 | slightly positive |
| Garlic powder | 0.1 | no contribution |
| | 0.5 | weakly positive |

**Claims**

1.  A process for producing edible matter having an enhanced flavour derived from incubating a microorganism capable of producing edible non toxic substances in an aqueous fermentation medium with an

11

$a_w$ of at least 0.97 in the presence of meat protein and a carbon containing source of energy, wherein said microorganism is cultivated in said aqueous fermentation medium for a period of 1 to 20 hours after finishing the logarithmic growth phase, i.e. after 0-20 hours in the stationary growth phase.

2. A process according to claim 1, wherein after sufficient fermentation the microorganism is inactivated without altering the character of the flavour obtained from the fermentation.

3. A process according to claim 2, wherein the inactivation is carried out by heating the fermentation broth during a period of time, t at a temperature T, wherein the values of t and T are equal to or lower than the values illustrated in the curve of figure 1.

4. A process according to claim 3, wherein the microorganism is inactivated during a period of time selected from between 14 and 30 seconds at a temperature equal to or lower than the value illustrated in the curve of figure 1 corresponding to said inactivation period.

5. A process according to claim 3 or 4, wherein the microorganism is inactivated during a period of time at a temperature equal to or lower than 75°C.

6. A process according to any of the previous claims, wherein the aqueous fermentation medium comprises from 2 to 3% by weight of meat or an extract thereof, preferably beef extract and from 1.5 to 2.5% by weight of a carbon containing source of energy.

7. A process according to any of the previous claims, wherein the carbon containing source of energy comprises at least one sugar, preferably selected from the group consisting of glucose, lactose and maltose.

8. A process according to any of the previous claims, wherein the microorganism is selected from a group comprising bacteria, yeasts and moulds, for example a yeast selected from one of the genera Candida and Hansenula, preferably a strain selected from either the species Candida utilis, such as the strain Candida utilis ATCC 26387 or CBS 7502, or selected from the species Debaryomyces hansenii such as the strain Debaryomyces hansenii CBS 7531.

9. A process according to any of the previous claims, wherein the cultivation is carried out under aerobic conditions.

10. A process according to claim 9, wherein the cultivation is carried out with an amount of oxygen corresponding to at least 10% of the saturation level of dissolved oxygen at atmospheric pressure.

11. A process according to any of the previous claims, wherein the cultivation is carried out at a temperature in the range of 20°C to 38°C, preferably in a range of 25°C to 35°C.

12. A process according to any of the previous claims, wherein the cultivation is carried out at a pH in the range of 4.5 to 6.0, preferably at about 5.0.

13. A process according to any of the previous claims, wherein the aqueous fermentation medium further comprises from 1.5 to 2.5% by weight of sodium chloride.

14. A process according to any of the previous claims, wherein the aqueous fermentation medium further comprises yeast extract, preferably in at least 0.5% by weight.

15. A process according to any of the previous claims, wherein the aqueous fermentation medium further comprises one or more amino acids selected from the group comprising glycine, isoleucine, asparagine, arginine, with preference for leucine, phenylalanine, cysteine, methionine and tryptophane.

16. A process according to any of the previous claims, wherein the aqueous fermentation medium further comprises at least one spice such as garlic powder and/or white pepper.

12

**17.** A process according to any of the previous claims, wherein the aqueous fermentation medium further comprises at least one hydrolysing enzyme such as papaine.

**18.** Edible matter with enhanced flavour obtainable through any of the processes according to any of the claims 1-17.

**19.** A flavouring substance comprising the edible matter according to claim 18.

**20.** A flavouring substance according to claim 19 exhibiting a sausage flavour.

**21.** A flavouring substance according to claim 19 or 20 exhibiting a fermented meat flavour.

**22.** A method for imparting flavour to foodstuffs or ingredients for foodstuffs, wherein a flavouring or aromatizing amount of the edible matter according to claim 18 or of the flavouring substance according to any of claims 19-21 is incorporated into the foodstuff or the ingredient for a foodstuff.

**23.** A foodstuff or an ingredient for a foodstuff obtained by the method for imparting flavour according to claim 22.

FIGURE I

14

European Patent
Office

EUROPEAN SEARCH REPORT

Application Number

EP    91 20 3382

Page 1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 357 812 (PHILLIPS PETROLEUM)<br><br>* claims *<br>--- | 1,8,22,<br>23 | A23L1/23<br>A23L1/318<br>C12N1/00 |
| A | EP-A-0 029 503 (STAUFFER CHEMICAL)<br>* claims *<br>--- | 1 | |
| A | EP-A-0 321 692 (KARL MÜLLER)<br>* claims *<br>--- | 1,8 | |
| A | US-A-3 193 391 (C.E.JANSEN ET AL.)<br>* claims *<br>--- | 1,8 | |
| D,A | DIE FLEISCHWIRTSCHAFT<br>vol. 67, no. 3, 1987,<br>pages 307 - 314;<br>F-K. LÜCKE ET AL.: 'Starter cultures for dry sausages and raw ham.Composition and effect'<br>* the whole document *<br>--- | 1 | |
| D,A | DIE NAHRUNG<br>vol. 30, no. 8, 1986,<br>pages 829 - 832;<br>E.MITEVA ET AL.: 'Sensory aroma and taste profiles of raw-dried sausages manufactured with a lipolitically active yeast culture'<br>* the whole document *<br>--- | 1 | TECHNICAL FIELDS SEARCHED (Int. Cl.5)<br><br>A23L<br>C12N |
| A | CHEMICAL ABSTRACTS, vol. 77, no. 25,<br>18 December 1972, Columbus, Ohio, US;<br>abstract no. 163192T, 'Improvement in the aroma of coocked suasages'<br>page 298 ; column 1 ;<br>* abstract *<br>& MYAS.IND.SSSR<br>no. 8, 1972, MOSCOW<br>pages 34 - 36;<br>V.PAL'MIN ET AL.:<br>--- | 1 | |

-/--

The present search report has been drawn up for all claims

| Place of search | Date of completion of the search | Examiner |
|---|---|---|
| THE HAGUE | 23 JULY 1992 | VAN MOER A.M.J. |

European Patent
Office

EUROPEAN SEARCH REPORT

Application Number

EP    91 20 3382
Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| E,X | EP-A-0 483 888 (UNILEVER)<br>* claims *<br><br>----- | 1,22,23 | |
| | | | **TECHNICAL FIELDS SEARCHED (Int. Cl.5)** |

The present search report has been drawn up for all claims

| Place of search | Date of completion of the search | Examiner |
|---|---|---|
| THE HAGUE | 23 JULY 1992 | VAN MOER A.M.J. |